# FASCICULE DE BREVET EUROPEEN

(11) **EP 2 515 840 B1**
(45) Date de publication et mention de la délivrance du brevet: **04.10.2017**
(21) Numéro de dépôt: 10807705.8
(22) Date de dépôt: 21.12.2010
(51) Int. Cl.: A61K 8/64, A61Q 19/00, A61Q 19/08, A61K 8/97

(54) **COMPOSITION COSMETIQUE ET/OU PHARMACEUTIQUE COMPRENANT UN EXTRAIT DE CAROUBE EN TANT QU'AGENT ACTIF ACTIVATEUR DE L'EXPRESSION DES AQUAPORINES**
KOSMETISCHE UND/ODER PHARMAZEUTISCHE ZUSAMMENSETZUNG ENTHALTEND EINEN JOHANNISBROTBAUM-EXTRAKT ALS AKTIVEN INHALTSSTOFF ZUR AKTIVIERUNG DER AQUAPORIN-EXPRESSION
COSMETIC AND/OR PHARMACEUTICAL COMPOSITION COMPRISING AN EXTRACT OF CAROB AS ACTIVE AGENT FOR ACTIVATING AQUAPORIN EXPRESSION

(30) Priorité: 24.12.2009 FR 0906346
(43) Date de publication de la demande: 31.10.2012
(73) Titulaire: ISP Investments Inc., Wilmington, DE 19805 (US)
(72) Inventeur: DAL FARRA, Claude, Kerhonkson, NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000855
(87) Numéro de publication internationale: WO 2011/077017

(56) Documents cités:
- EP-A1- 0 237 398
- WO-A2-2009/101503
- FR-A1- 2 934 779
- PARRADO ET AL: "Production of a carob enzymatic extract: Potential use as a biofertilizer", BIORESOURCE TECHNOLOGY, ELSEVIER BV, GB LNKD- DOI:10.1016/J.BIORTECH.2007.05.029, vol. 99, no. 7, 8 février 2008 (2008-02-08), pages 2312-2318, XP022472753, ISSN: 0960-8524 cité dans la demande
- LU-JIA HAN ET AL.: "In vitro antioxidant activity of protein hydrolysates prepared from corn gluten meal", J. SCI. FOOD AGRIC., vol. 88, 20 mai 2008 (2008-05-20), pages 1660-1666, XP002594813,

## Description

### Domaine de l'invention

La présente invention se situe dans le domaine cosmétique et pharmaceutique, et plus particulièrement dans le domaine de la dermatologie. L'invention concerne une composition cosmétique et/ou pharmaceutique comprenant, dans un milieu physiologiquement adapté, comme agent actif activateur de l'expression des aquaporines, un extrait peptidique de caroube *(Ceratonia siliqua L.)* obtenu après hydrolyse et contenant des peptides de poids moléculaire inférieur à 5 kDa et ayant une teneur en polyphénols inférieure à 1 %. La présente invention a également pour objet l'utilisation d'un extrait peptidique de caroube *(Ceratonia siliqua L.)* en tant qu'agent actif activateur de l'expression des aquaporines dans une composition cosmétique pour améliorer l'hydratation et la fonction barrière de l'épiderme et pour stimuler la régénération cutanée. L'agent actif peut être utilisé seul ou en association avec d'autres agents actifs. L'invention est également relative à l'utilisation de ce nouvel agent actif pour la réalisation d'une composition pharmaceutique, et notamment dermatologique, par voie orale ou topique, destinée à réguler et/ou stimuler l'activité des aquaporines et traiter ainsi les pathologies impliquant une sécheresse pathologique de la peau ou des muqueuses, telles que la xérose ou l'eczéma, les symptômes de bouche sèche ou la sécheresse de l'oeil. L'invention porte encore sur un procédé de traitement cosmétique destiné à prévenir ou à lutter contre la sécheresse de la peau et des muqueuses et les manifestations du vieillissement cutané, selon lequel on applique l'extrait de caroube activateur de l'expression des aquaporines ou une composition le contenant sur les zones à traiter.

### Arrière plan de l'invention

La peau est un organe vital composé de plusieurs couches (derme, couches prolifératives et *stratum corneum*) qui recouvre toute la surface du corps et assure essentiellement une fonction de barrière vis-à-vis du milieu extérieur. Cette fonction barrière repose en particulier sur la qualité de l'épiderme qui dépend notamment de l'état du *stratum corneum* et de l'équilibre entre la prolifération et la différenciation des kératinocytes épidermiques. La qualité et le bon fonctionnement de la peau sont étroitement liés à la teneur en eau des différentes couches de l'épiderme. Ainsi, dans un épiderme normal, les couches prolifératives contiennent environ 70 % d'eau, alors que le *stratum corneum* en contient seulement 10 à 15 %.

L'hydratation du *stratum corneum* est la résultante de trois facteurs : l'approvisionnement en eau à partir du derme, la perte en eau vers le milieu extérieur et la capacité du *stratum corneum* à fixer les molécules d'eau.

La régulation de la distribution en eau se fait par des hormones (aldostérone, hormones sexuelles), le pH ou les variations osmotiques. Les membranes cellulaires sont de nature hydrophobes et donc peu perméables à l'eau, mais il existe des canaux hydriques, sorte de pores facilitant le passage de l'eau et de certains solutés.

Les aquaporines sont une classe de protéines transmembranaires transporteurs d'eau et de petites molécules en solution, telles que le glycérol et l'urée, qui facilitent le transport de l'eau dans les épithéliums et les endothéliums.

La fonction déterminante des aquaporines dans les épithéliums impliqués dans le transport de l'eau ou des solutés, tels que le rein, a été bien étudiée (Deen et al., 1994). De même, les aquaporines ont été rapidement identifiées dans les glandes exocrines productrices de la salive ou des pleurs. Toutefois, la découverte d'aquaporines de type 3, ou AQP3, dans l'épiderme humain, et plus précisément dans la membrane plasmique des kératinocytes des couches prolifératives de l'épiderme (SOUGRAT R. et al., J. Invest. Dermatol., 2002), a mis en lumière l'importance des flux hydriques régulés dans la peau. Les AQP3 sont capables de transporter l'eau et le glycérol, ce dernier jouant un rôle important dans la constitution du film hydrolipidique de surface ainsi que dans le maintien de la souplesse et des qualités sensorielles du *stratum corneum.*

L'importance des AQP3 a été démontrée chez la souris. En effet, l'inactivation du gène provoque l'apparition de multiples déficiences de la peau, telles qu'un faible taux d'hydratation, une fonction barrière inefficace, un temps de régénération tissulaire augmenté et une diminution de l'élasticité (Ma T. et al., J. Mol. Biol., 2002). On a découvert depuis que l'aquaporine 3 est exprimée dans les fibroblastes dermiques où elle intervient dans la migration de ces cellules lors de la régénération des plaies (Cao C. et al., Biochem J., 2006). D'autre part, les aquaporines jouent un rôle dans la fonction barrière en régulant positivement l'établissement des liaisons et communications cellulaires de type tight junction (Kawedia J. et al., PNAS 104(9), 2007).

L'hydratation et la teneur en AQP3 des kératinocytes sont étroitement liées. Ainsi, l'augmentation d'AQP3 dans la peau provoque une meilleure hydratation de l'épiderme (Dumas M., J. Drugs Dermatol., Jun6, 2007).

Les pertes cutanées en eau peuvent avoir plusieurs origines, héréditaires, acquises ou liées à l'environnement. Dans un environnement très sec, les pertes en eau par évaporation à partir du *stratum corneum* sont significatives et peuvent excéder le taux de remplacement par diffusion transcellulaire.

Au cours du vieillissement cutané, la peau devient sèche. Ainsi, il est très souvent constaté chez les sujets âgés, et notamment de plus de 50 ans, la manifestation d'une xérose ou d'une sécheresse des muqueuses liée à une moindre sécrétion de sébum, à des modifications hormonales ou à des ralentissements du flux hydrique à travers l'épiderme. La peau est alors le siège de démangeaisons et de tiraillements, deux symptômes caractéristiques d'une peau sèche. Parmi les pathologies acquises se traduisant par une sécheresse de la peau, on peut citer la xérose induite par la photo-chimiothérapie et l'eczéma. Parmi les pathologies acquises provoquant une sécheresse de la bouche, ou xérostomie, on peut citer le syndrome de Sjogren ou la radiothérapie du cou. Enfin, parmi les pathologies impliquant une sécheresse des muqueuses, on peut citer les sécheresses oculaires ou vaginales.

Une première alternative de traitement des peaux sèches consiste à administrer par voie topique des produits destinés à restaurer la barrière cutanée, tels que des agents humectants capables de fixer l'eau, parmi lesquels on peut citer l'urée et l'acide lactique qui entrent dans la composition du NMF (Natural Moisturizer Factor ; dérivés protéolytiques de la filaggrine), des agents filmogènes destinés à retenir l'eau, ou encore des agents capables de reconstruire la barrière cutanée (squalènes, céramides acides gras). Toutefois, ces produits ont une action superficielle qui ne corrige pas les défauts biologiques d'une peau souffrant de déshydratation chronique.

Dans ce contexte, les propriétés particulières des aquaporines en font des cibles biologiques possibles pour améliorer l'hydratation de la peau et diminuer les signes de sécheresse cutanée. Ainsi, le brevet FR 2 801 504 décrit l'augmentation d'AQP3 dans la peau par un extrait de plantes A*juga turkestanica* et a permis d'améliorer l'hydratation de la peau. Un extrait de grenade a également été décrit comme agent actif par voies orale et topique pour stimuler l'activité des aquaporines et réguler les mouvements d'eau et de glycérol dans les tissus (FR 2 874 502). Un extrait total de pulpe de caroube, contenant à la fois des protéines, des polyphénols et des glucides et son utilisation pour hydrater la peau, ont été décrits dans le brevet français FR 2 905 857 sans que l'on puisse présager du mécanisme d'action d'un tel extrait.

D'autre part, un extrait protéique de germe de caroube obtenu par hydrolyse et destiné à produire un fertilisant facilement assimilable par les plantes a déjà été décrit (J. Parrado et al., Bioresource Technology 99, 2008). L'extrait décrit dans la publication est riche en peptides de bas poids moléculaire, mais également en sucres et en phytohormones. Or, les phytohormones sont des composés polyphénoliques modulateurs de la croissance des plantes, mais considérés comme des perturbateurs endocriniens pour l'homme car ils sont souvent susceptibles d'altérer des fonctions telles que la croissance, le développement, le comportement ou la production. La présence de ce type de composés n'est donc pas souhaitable dans un extrait destiné à une application cosmétique ou pharmaceutique.

La présente invention décrit un extrait peptidique de germe de caroube contenant des peptides de poids moléculaire inférieur à 5 KDa et présentant un taux de polyphénols très faible (inférieur à 1 %) et un taux de sucre inférieur à 15%. Cette composition chimique procure une grande innocuité à l'extrait et permet d'obtenir une activité moléculaire mieux ciblée. Les inventeurs ont ainsi mis en évidence que l'extrait de l'invention provoquait une activation de l'expression des aquaporines et en particulier de l'aquaporine 3.

### Exposé de l'invention

Le premier objet de la présente invention est un extrait peptidique de caroube (*Ceratonia siliqua L.*), activateur de l'expression des aquaporines, caractérisé en ce qu'il est obtenu après hydrolyse et en ce que les composés de nature peptidique sont des peptides de poids moléculaire inférieur à 5 kDa et que sa teneur en polyphénols est inférieure à 1 %.

Les inventeurs ont en effet mis en évidence que cet extrait peptidique de caroube, lorsqu'il est appliqué sur la peau, améliore l'hydratation et la fonction barrière de l'épiderme et des muqueuses et stimule la régénération cutanée. Ces propriétés ont été démontrées par une protection du tissu cutané et une diminution de l'apoptose à la suite d'un stress hydrique.

On entend par « agent actif capable de prévenir et de lutter contre la sécheresse de la peau et des muqueuses et les manifestations du vieillissement cutané», toute substance capable d'améliorer l'hydratation et la fonction barrière de l'épiderme et des muqueuses ou de diminuer l'apoptose dans des cellules ou des tissus soumis à un stress hydrique.

On entend par « extrait peptidique », un mélange dilué de composés majoritairement représentés par des peptides, en solution dans un large volume d'eau ou d'autres solvants, polaires ou un mélange de ces solvants.

On entend par « application topique », le fait d'appliquer ou d'étaler l'agent actif selon l'invention, ou une composition le contenant, à la surface de la peau ou d'une muqueuse.

On entend par « physiologiquement acceptable », que l'agent actif selon l'invention, ou une composition le contenant, est approprié pour entrer en contact avec la peau ou une muqueuse sans provoquer de réactions de toxicité ou d'intolérance.

Par l'expression « biologiquement actif », on entend « qui possède une activité *in vivo* ou *in vitro* caractéristique de l'activité de l'agent actif selon l'invention ».

On entend par « composés de nature peptidique », les fragments de protéines et les peptides présents dans l'extrait peptidique selon l'invention.

L'activité biologique caractéristique selon l'invention est définie *in vitro* par la capacité de l'agent actif à activer l'expression des aquaporines, soit par l'augmentation de la synthèse protéique de l'aquaporine (par modulation directe ou indirecte de l'expression génique de l'aquaporine), soit par d'autres processus biologiques tels que la stabilisation de la protéine aquaporine ou encore la stabilisation des transcrits d'ARN messager.

Préférentiellement, selon la présente invention, l'aquaporine est l'aquaporine 3, ou AQP3, présente dans les membranes des kératinocytes.

L'agent actif selon l'invention peut être obtenu par extraction de protéines d'origine végétale, suivie d'une hydrolyse contrôlée qui libère les composés de nature peptidiques biologiquement actifs.

L'utilisation d'extraits peptidiques, et en particulier d'extraits peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provoquant pas de réactions allergiques en dermato-cosmétique.

De très nombreuses protéines de plantes sont susceptibles de contenir des peptides bioactifs au sein de leur structure. L'hydrolyse ménagée permet de dégager ces composés de nature peptidique. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés de nature peptidique ensuite.

Pour réaliser l'extraction, on utilise les graines de caroube (plante du genre Cératonia). La caroube est cultivée dans les pays méditerranéens et la fraction endosperme de sa graine, est utilisée dans l'industrie alimentaire sous la dénomination "gomme de caroube". Le germe est la partie la plus riche en protéine de la graine et peut être aisément isolé.

Toute méthode d'extraction ou de purification connue de l'homme du métier peut être utilisée afin de préparer l'extrait selon l'invention.

Dans une première étape, les germes contenus dans les graines sont broyés afin d'obtenir une poudre ou farine. La poudre ainsi obtenue peut être préalablement traitée par une cellulase afin de favoriser l'élimination des sucres, et en particulier des polysaccharides insolubles.

On réalise ensuite l'extraction des protéines du germe suivant le procédé classique (Osborne, 1924) modifié ; le broyat de germe de caroube est mis en suspension dans une solution alcaline contenant un produit adsorbant de type polyvinylpolypyrrolidone (PVPP) insoluble (0,01 -20 %) ; en effet, il a été observé que les opérations d'hydrolyses et de purifications ultérieures étaient facilitées par ce moyen. En particulier, la concentration en substances de type phénoliques, interagissant avec les protéines, se trouve nettement réduite. Les protéines peuvent ensuite être précipitées en faisant varier la force ionique ou en acidifiant le milieu, ce qui permet d'éliminer les composants solubles et les acides nucléiques. Le précipité est ensuite lavé à l'aide d'un solvant organique tel que, par exemple, l'éthanol ou le méthanol, puis le solvant est évaporé par séchage sous vide. Le précipité riche en protéines est remis en solution dans l'eau ou un autre solvant et constitue alors une forme plus purifiée de l'extrait.

L'extraction peut également être réalisée en milieu neutre ou acide toujours en présence de polyvinylpolypyrrolidone. Après une étape de filtration, l'étape de précipitation s'effectue alors à l'aide d'un agent classique de précipitation tel que les sels (chlorure de sodium, sulfate d'ammonium) ou un solvant organique (alcool, acétone). Le précipité obtenu peut être séparé des agents de précipitation par dialyse après remise en solution dans de l'eau ou un autre solvant.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques parmi lesquelles on peut alors citer les endoprotéases d'origine végétale (papaïne, bromelaïne, ficine).

Pour les mêmes raisons que précédemment, c'est-à-dire l'élimination des substances polyphénoliques, une quantité de polyvinylpolypyrrolidone peut être additionnée au milieu réactionnel lors de cette étape d'hydrolyse ménagée. L'extrait obtenu peut être encore purifié afin de sélectionner les composés de nature peptidiques de bas poids moléculaires. Le fractionnement peut s'effectuer avantageusement par ultrafiltration et/ ou par une méthode de type chromatographique.

On procède ensuite à une phase de dilution dans de l'eau ou dans tout mélange de solvants contenant de l'eau. Ainsi, l'agent actif selon l'invention est avantageusement solubilisé dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'agent actif dilué est ensuite stérilisé par ultrafiltration.

Après cette dilution, on obtient un extrait peptidique caractérisé par un poids sec de 2 à 5 g/Kg, une concentration en composés de nature peptidique de 1 à 10 g/l, préférentiellement de 1,5 à 3,5 g/l et une concentration en sucres de 0,05 à 1 g/l, préférentiellement de 0,1 à 0,3 g/l et une concentration en polyphénols inférieure à 1 % par rapport au poids sec. Les solvants utilisés sont physiologiquement acceptables et classiquement utilisés par l'homme du métier choisis parmi le glycérol, l'éthanol, le propanediol, le butylène glycol, le dipropylène glycol, les diglycols éthoxylés ou propoxylés, les polyols cycliques ou tout mélange de ces solvants.

L'extrait obtenu selon l'invention est analysé qualitativement et quantitativement, selon les techniques classiques bien connues de l'homme du métier, pour ses caractéristiques physico-chimiques et sa teneur en composés de nature protéique et peptidique.

L'extrait obtenu est composé de peptides de poids moléculaire inférieur à 5 kDa et est caractérisé par une concentration en sucres inférieure à 15 % et une concentration en polyphénols inférieure à 1 % par rapport au poids sec.

Le deuxième objet de la présente invention est une composition comprenant, dans un milieu physiologiquement acceptable, en tant qu'agent actif activateur de l'expression des aquaporines, l'extrait peptidique de germe de caroube selon l'invention, à une concentration comprise entre 0,0001 % et 20 % du poids total de la composition, préférentiellement à une concentration comprise entre 0,05 % et 5 % du poids total de la composition et comprenant au moins un autre agent actif choisi parmi le glycérol, la vitamine C, la vitamine E, le coenzyme Q10, les rétinoïdes.

L'agent actif peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites.

Ces compositions pourront notamment se présenter sous forme d'une solution aqueuse, hydro-alcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

La composition utilisable selon l'invention pourra être appliquée par toute voie appropriée, notamment orale, parentérale ou topique externe, et la formulation des compositions sera adaptée par l'homme du métier.

Avantageusement, les compositions selon l'invention se présentent sous une forme adaptée à l'application par voie topique. Ces compositions doivent donc contenir un milieu physiologiquement acceptable, c'est-à-dire compatible avec la peau et les phanères, et couvrent toutes les formes cosmétiques ou dermatologiques.

Il est bien évident que l'invention s'adresse aux mammifères en général, et plus particulièrement aux êtres humains.

Il est bien entendu que l'agent actif selon l'invention peut être utilisé seul ou bien en association avec d'autres agents actifs. Ainsi, les compositions utilisables selon l'invention contiennent en outre divers agents actifs favorisant l'action dudit extrait peptidique. Par exemple, la composition selon l'invention peut associer un autre agent actif hydratant tel que le glycérol. Cette association est particulièrement avantageuse et procure une efficacité d'hydratation optimale, comparée à l'utilisation du glycérol seul.

On peut également citer, de manière non limitative, les ingrédients suivants : des agents cicatrisants, anti-âge, antirides, apaisants, anti-radicalaires, anti-LTV, des agents stimulant la synthèse de macromolécules dermiques ou le métabolisme énergétique, des agents hydratants, antibactériens, antifongiques, anti-inflammatoires, anesthésiques, des agents modulants la différenciation, la pigmentation ou la dépigmentation cutanée, des agents stimulant la pousse des ongles ou des cheveux.

Préférentiellement, on utilisera un agent présentant une activité antiride, tel qu'un agent anti-radicalaire ou antioxydant, ou un agent stimulant la synthèse de macromolécules dermiques, agent stimulant le métabolisme énergétique, inhibiteur de métallo-protéinase.

Par exemple, il peut être ajouté d'autres principes actifs ayant une action anti-radicalaire ou antioxydante, choisis parmi la vitamine C, la vitamine E, le coenzyme Q10 et les extraits polyphénoliques de plantes, les rétinoïdes.

La composition selon l'invention peut encore associer au principe actif selon l'invention, d'autres principes actifs stimulant les synthèses des macromolécules dermiques (laminine, fibronectine, collagène), par exemple, le peptide de collagène commercialisé sous le nom « Collaxyl^{®} » par la société Vincience.

L'invention a pour troisième objet une composition pharmaceutique comprenant, dans un milieu physiologiquement acceptable, l'extrait peptidique selon l'invention à titre de médicament. La composition pharmaceutique selon l'invention permettra de réguler et/ou stimuler l'activité des aquaporines. La composition pharmaceutique peut être destinée à prévenir ou à traiter les sécheresses pathologiques de la peau ou des muqueuses.

La composition pharmaceutique peut également être destinée à prévenir ou traiter les pathologies provoquées par les dysfonctionnements des aquaporines dans la peau et les muqueuses, tels que l'eczéma, la xérose, les dermatites atopiques, les sécheresses buccales, oculaires ou vaginales.

Selon cette forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirop, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres principes actifs pharmaceutiques, des huiles essentielles, des vitamines, des acides gras essentiels, etc.

L'invention a pour quatrième objet l'utilisation cosmétique de l'extrait peptidique de germe de caroube (*Ceratonia siliqua L.*) selon l'invention, en tant qu'agent actif hydratant, améliorant la fonction barrière et régénérant.

Ainsi selon cet objet de l'invention, l'extrait peptidique de germe de caroube est utilisé dans une composition cosmétique pour améliorer l'hydratation de la peau et prévenir ou lutter contre la sécheresse de la peau et des muqueuses.

Selon ce même objet de l'invention, l'extrait peptidique de germe de caroube est utilisé dans une composition cosmétique pour améliorer la fonction barrière de l'épiderme.

Selon cette forme de l'invention, l'extrait peptidique de germe de caroube pourra également être utilisé dans une composition cosmétique pour protéger la peau et les muqueuses contre tous types d'agressions extérieures.

On entend par l'expression « agression extérieure », les agressions que peut produire l'environnement. A titre d'exemple, on peut citer des agressions telles que la pollution, les rayonnements ultraviolets, ou encore les produits à caractère irritant tels que les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sécheresse de l'atmosphère est également une cause importante de dessèchement cutané.

L'extrait peptidique de germe de caroube pourra ainsi être utilisé, en tant qu'agent actif, dans une composition cosmétique pour prévenir les dommages causés à la peau par une exposition au soleil ou un environnement desséchant.

Les agressions que subissent la peau et les phanères peuvent également être dues à un déséquilibre du gradient électrochimique à travers la membrane cellulaire, ce qui peut aboutir à des variations importantes de la pression osmotique, et cela peut avoir pour conséquence des chocs osmotiques et donc la lyse des cellules.

Or, les inventeurs ont démontré, d'une manière surprenante, que l'agent actif selon l'invention protège les cellules contre ces chocs osmotiques.

La peau peut également être agressée par des traitements tels que le rasage. Avantageusement, l'invention a pour objet l'utilisation dans une composition cosmétique, d'un agent actif tel que décrit précédemment, l'agent actif, ou la composition le contenant, étant destiné à prévenir ou à traiter les dommages causés à la peau par le rasage.

Selon cette forme de l'invention, l'extrait peptidique de germe de caroube pourra également être utilisé dans une composition cosmétique pour stimuler la régénération cutanée.

Selon cette forme de l'invention, l'extrait peptidique de germe de caroube pourra également être utilisé dans une composition cosmétique pour lutter de manière préventive et/ou curative contre les manifestations du vieillissement cutané et, plus spécifiquement, pour lutter contre et/ou de prévenir le vieillissement photo-induit (photo-vieillissement). Par manifestations cutanées du vieillissement, on entend toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV). L'agent actif selon l'invention, ou la composition le contenant, permettra de lutter, en particulier, contre la perte d'élasticité et de fermeté de la peau.

L'invention consiste encore en un procédé de traitement cosmétique destiné à améliorer l'aspect de la peau et prévenir ou à lutter contre la sécheresse de la peau et des muqueuses, selon lequel on applique une composition selon l'invention sur les zones à traiter.

L'invention consiste encore en un procédé de traitement cosmétique destiné à prévenir et/ou à lutter contre les signes cutanés du vieillissement et/ou du photo-vieillissement, selon lequel on applique une composition selon l'invention sur les zones à traiter.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Préparation d'un extrait peptidique de caroube (Ceratonia siliqua L.)

Le germe de caroube (*Ceratonia siliqua L.)* sous forme de poudre est mis en solution dans 70 volumes d'eau et le pH est ajusté à une valeur comprise entre 4,5 et 5,5.

Afin d'éliminer les sucres insolubles, une hydrolyse à l'aide d'une cellulase est réalisée. Pour cela, 2% de celluclast CL® (novozym) et 2% de POLYCLAR® 10 (polyvinylpyrrolidone - PVPP - insoluble) sont ajoutés dans le milieu réactionnel. Le milieu réactionnel est ensuite chauffé deux heures à 50°C puis désactivé une heure à 80°C. Une étape de filtration permet d'écarter le filtrat riche en carbohydrates pour ne conserver que le résidu solide.

Ce dernier est caractérisé par une teneur en protéine comprise entre 45 et 50% et un taux de sucres compris entre 20 et 30%.

Le résidu sec ainsi obtenu est mis en solution dans 100 volumes d'eau en présence de 2% de POLYCLAR® 10. Le mélange est ajusté à un pH compris entre 8,0 et 8,5 avec une solution aqueuse de soude à 2 M.

Afin d'améliorer l'extraction des protéines, une première hydrolyse est réalisée à l'aide de 2% d'Alcalase® (novozym). L'hydrolyse est obtenue après 2 heures, sous agitation, à 55°C. On procède à l'inactivation de l'enzyme en chauffant la solution à 80°C pendant 2 heures. Après désactivation, le mélange réactionnel est filtré et le filtrat est recueilli. Il s'agit de l'extrait protéique intermédiaire de germe de caroube.

A ce stade de la préparation, les composés de nature peptidique et protéique de ce filtrat sont caractérisés par électrophorèse sur gel de polyacrylamide (gels NuPAGE® Bis-Tris Pre-cast, Invitrogen). Pour cela, le filtrat est chauffé à 70°C pendant 10 minutes dans des conditions réductrices dénaturantes dans un tampon de préparation d'échantillon NuPAGE® LDS. Une solution de NuPAGE® antioxydante est ajoutée dans la cuve intérieure (cathode) pour éviter que les protéines réduites ne se réoxydent durant l'électrophorèse. La migration des protéines est réalisée dans du tampon de migration NuPAGE® MES en présence d'un standard de poids moléculaire (SeeBlue Plus2). La coloration des protéines est effectuée à l'aide de Bleu de Coomassie® R-250. Le profil protéique ainsi obtenu montre que les composés de nature peptidique et protéique du filtrat ont des poids moléculaires compris entre 50 et 10 kDa.

L'extrait protéique intermédiaire de germe de caroube est ensuite mis en solution dans 100 volumes d'eau en présence de 2% de POLYCLAR® 10. Le mélange est ajusté à un pH compris entre 4 et 5 avec une solution aqueuse d'acide chlorhydrique 1 M.

Une étape d'hydrolyse des protéines est alors réalisée à l'aide d'une endoprotéase. Pour cela, de la bromélaïne à 2% est ajoutée dans le milieu réactionnel. L'hydrolyse est obtenue après 2 heures d'agitation à 50°C. On procède à l'inactivation de l'enzyme en chauffant la solution à 80°C pendant 2 heures.

La purification de l'extrait ainsi obtenu se poursuit par des filtrations successives à l'aide de filtres-plaques Seitz-Orion de porosité décroissante (jusqu'à 0,2 µm) afin d'obtenir une solution brillante et limpide. Après cette série de filtration, l'extrait de germe de caroube est caractérisé par un poids sec de 20-25 g/kg, un taux de protéines de 10-15 g/l, un taux de sucres de 5-6 g/l, un taux d'acides aminés de 1-2 g/l et un taux de polyphénols totaux de 0,5-1 g/l. Les protéines sont dosées par une méthode colorimétrique spécifique (méthode de Lowry)

Le profil protéique de cet extrait est analysé par gel d'électrophorèse. Dans les mêmes conditions que décrites précédemment, on observe 2 grandes familles de protéines : la 1ère famille, minoritaire, correspond à des protéines de poids moléculaire de 25 à 20 kDa et la 2ème famille, très majoritaire, correspond à des protéines de poids moléculaire inférieur à 5kDa.

Cet extrait est alors purifié en éliminant les protéines de poids moléculaire supérieur à 5 kDa par des étapes de filtrations à flux tangentiel. Pour cela, l'extrait de germe de caroube est pompé sous pression à travers un support Pellicon® équipée de cassette Pellicon® 2 Biomax 30 kDa. Le 1er filtrat obtenu est récupéré pour être de nouveau filtré à travers une autre cassette Pellicon® 2 Biomax 5 kDa.

En fin de purification, on obtient un extrait de germe de caroube jaune-orangé, brillant et limpide. Il est caractérisé par un poids sec de 8-9 g/kg, une teneur en protéines de 6-7 g/l, un taux de sucres entre 0,3 et 0,5 g/l et un taux de polyphénols totaux inférieur à 0,1 g/l.

On procède alors à une phase de dilution dans un mélange eau-glycérol afin d'obtenir un extrait peptidique caractérisé par un poids sec de 2 à 5 g/Kg, une concentration en composés de nature peptidique de 1,5 à 3,5 g/l, une concentration en sucres de 0,1 à 0,3 g/l (soit inférieure à 15 %) et une concentration en polyphénols inférieure à 1 %.

Cet extrait purifié et dilué correspond à l'extrait peptidique de germe de caroube selon l'invention. Il est caractérisé par le fait que les composés de nature peptidique sont des peptides de poids moléculaires inférieur à 5 KDa, que la teneur en polyphénols est inférieure à 1 %.

Cette solution est alors analysée en chromatographie liquide haute pression à l'aide d'un appareil HP1100 piloté par le logiciel ChemStation. La colonne utilisée lors de l'élution de l'extrait de caroube est une Nucleosil® 300-5 C4 MPN (125 x 4 mn). Cette colonne permet de chromatographier des protéines ayant des poids moléculaires de 0.2 à 25 kDa (selon un gradient de solvants approprié). Dans ces conditions chromatographiques, il a été isolé plusieurs fractions peptidiques. Ces diverses fractions ont été analysées par spectrométrie de masse afin d'identifier leurs pics moléculaires. La détermination de la composition en acides aminés a également été réalisée. Celle-ci est obtenue après hydrolyse acide et identification par chromatographie liquide haute pression à l'aide d'une pré-dérivation au PICT (phenylisothiocyanate).

### Exemple 2 : Evaluation de l'effet protecteur de l'agent actif selon l'exemple 1 vis-à-vis d'un choc osmotique

Le but de cette étude est de déterminer l'effet protecteur de l'agent actif selon l'exemple 1 vis-à-vis de kératinocytes humains normaux soumis à un choc osmotique provoqué par le sorbitol. L'évaluation de l'état cellulaire s'effectue ensuite par un test de viabilité au MTT.

### Protocole :

Des kératinocytes humains normaux en culture ont été mis en présence de l'agent actif selon l'exemple 1 à 0,5 %, 1 % et 3 %, 24 heures avant et pendant le choc osmotique. Les conditions de culture hypertoniques sont réalisées en ajoutant du sorbitol à 250 mM au milieu de culture pendant 24 heures. Des contrôles non traités sont réalisés.

Des tests de viabilité cellulaire ont ensuite été réalisés par la technique au MTT. L'agent MTT (3-[4, 5-diméthylthiazol-2-yl]-2, 5-diphényltétrazolium bromide) est utilisé pour évaluer la viabilité cellulaire. Les kératinocytes sont incubés dans une solution contenant 0,1 mg/ml de MTT (3-[4, 5-diméthylthiazol-2-yl]-2, 5-diphényltétrazolium bromide). Ce composé est absorbé par les cellules vivantes puis métabolisé par les enzymes mitochondriales (succinate déshydrogénase) en un composé bleu violet, le formazan, qui se présente sous forme de cristaux violets insolubles en milieu aqueux.

Les cristaux de formazan sont solubilisés dans du DMSO, ils donnent une densité optique (D.O.) proportionnelle au nombre de cellules vivantes présentes dans l'échantillon. Des mesures de densité optique ont ensuite été réalisées pour chaque échantillon étudié (la D.O. se lisant à 540 nm). La D.O. est alors directement proportionnelle à l'activité enzymatique ainsi qu'au nombre de cellules vivantes.

### Résultats :

Les résultats obtenus montrent une augmentation de la viabilité, dose-dépendante, des cellules soumises au choc osmotique lorsque les cellules sont traitées avec l'agent actif selon l'exemple 1, en comparaison avec les cellules non traitées. L'augmentation de viabilité est supérieure à 10 % lorsque les cellules sont traitées avec l'agent actif selon l'exemple 1 à 3 %,

### Conclusions :

L'agent actif selon l'exemple 1 protège efficacement les kératinocytes humains normaux soumis à un choc osmotique.

### Exemple 3 : Evaluation de l'effet protecteur de l'agent actif selon l'exemple 1 vis-à-vis d'une déshydratation cutanée induite

Le but de cette étude est de déterminer l'effet protecteur de l'agent actif selon l'exemple 1, vis-à-vis de cultures d'épiderme *ex vivo* soumises à un stress par une déshydratation cutanée induite.

### Protocole :

Des biopsies de peau humaine sont débarrassées de leur couche cornée par arrachage à l'aide de ruban adhésif (tape stripping) puis maintenues en culture *ex vivo,* traitées avec une solution à 1 %, d'agent actif selon l'exemple 1, pendant 24 heures. L'absence de couche cornée induit une déshydratation importante. Des coupes et des colorations histologiques hématoxyline-éosine (H&E) permettent d'évaluer la qualité des structures cutanées.

### Résultats :

L'observation des coupes de peau montrent une nette diminution des signes de stress cellulaire et une meilleure préservation des structures cutanées dans les biopsies de peau traitées par l'agent actif selon l'exemple 1, comparées aux biopsies de peau non traitées. D'autre part, l'aspect des kératinocytes de l'assise basale montre un effet régénérant de l'agent actif selon l'exemple 1.

### Conclusions :

L'agent actif selon l'exemple 1 protège efficacement la peau du stress induit par la déshydratation. D'autre part, l'agent actif selon l'exemple 1 permet une régénération de l'épiderme.

### Exemple 4 : Mise en évidence de l'effet activateur de l'agent actif selon l'exemple 1 sur l'expression des claudines et de la kératine K10

Le but de cette étude est de déterminer l'influence de l'agent actif selon l'exemple 1 sur l'expression des claudines et de la kératine K10. Les claudines sont les principales protéines transmembranaires des structures d'adhésion intercellulaires nommées tight-junction qui jouent un rôle dans la communication cellulaire et la cohésion épidermique. La kératine K10 est une kératine spécifique des couches épidermiques différenciées (*stratum granulosum*) et intervient dans la fonction barrière de la peau. La quantité de protéine a été évaluée par immunofluorescence sur des coupes de peau humaine.

### Protocole :

Des échantillons de peau humaine sont mis en culture à l'interface air/liquide. Une solution à 1 % d'agent actif selon l'exemple 1 est appliquée topiquement, puis les échantillons sont incubés durant 24 heures.

Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes.

L'immunomarquage de la Claudin-1 est réalisé à l'aide d'un anticorps spécifique de la claudin-1 (anticorps claudin-1: rabbit polyclonal, ref: Ab15098, Abcam, dilution 1/200^{ème}), puis d'un anticorps secondaire, couplé à un marqueur fluorescent (alexa Fluor 488 donkey anti-rabbit IgG, A21206, Molecular Probes, 1/1000^{ème}).

L'immunomarquage de la Kératine 10 est réalisé à l'aide d'un anticorps spécifique de la kératine 10 (anticorps K10: mouse monoclonal, ref: LHP1, Novocastra, dilution 1/50^{ème}), puis d'un anticorps secondaire, couplé à un marqueur fluorescent (alexa Fluor 488 donkey anti-mouse IgG, A21202, Molecular Probes, 1/1000^{ème}).

Les coupes de peau sont alors examinées au microscope à Epi-fluorescence (Nikon Eclipse E600 microscope).

### Résultats :

Les observations microscopiques montrent une fluorescence plus forte dans les peaux traitées par l'agent actif selon l'exemple 1 à 1 %, en particulier les couches supérieures de l'épiderme aussi bien pour la protéine claudin-1 que pour la kératine K10.

### Conclusions :

L'agent actif selon l'exemple 1 stimule fortement l'expression des claudines, en particulier dans les couches supérieures de l'épiderme. De même, l'agent actif selon l'exemple 1 stimule fortement l'expression de la kératine K10, et plus généralement les fonctions barrières de l'épiderme.

### Exemple 5 : Mise en évidence de l'effet stimulant de l'agent actif selon l'exemple 1 sur l'expression d'aquaporines

Le but de cette étude est de déterminer l'influence de l'agent actif selon l'exemple 1 sur l'expression de l'aquaporine 3 dans des échantillons de peau *ex vivo.*

### Protocole :

Des échantillons de peau humaine sont mis en culture à l'interface air/liquide. Une solution à 1 % d'agent actif selon l'exemple 1 est appliquée topiquement, puis les échantillons sont incubés durant 24 heures ou 48 heures.

Ces échantillons de peau sont ensuite fixés avec du formaldéhyde puis inclus dans la paraffine. Des coupes de 2 à 3 µm sont alors réalisées. L'immunomarquage est effectué après différentes étapes de lavage et d'incubation de ces coupes. L'immunomarquage est réalisé à l'aide d'un anticorps polyclonal spécifique de l'aquaporine 3 (Anti-aquaporin 3 (C- 18): goat polyclonal, sc-9885, Santa Cruz, dilution 1/100^{ème}), puis d'un anticorps secondaire, couplé à un marqueur fluorescent (Alexa-fluor 488 donkey anti-goat Ig G, Molecular Probes, dilution 1/1000^{ème}). Les coupes de peau sont alors examinées au microscope à Epi-fluorescence (Nikon Eclipse E600 microscope).

### Résultats :

Les observations microscopiques montrent une fluorescence plus forte dans les peaux traitées par l'agent actif selon l'exemple 1 à 1 %, en particulier dans les couches supérieures de l'épiderme par rapport au contrôle non traité.

### Conclusions :

L'agent actif selon l'exemple 1 stimule l'expression de l'aquaporine 3, en particulier dans les couches supérieures de l'épiderme.

### Exemple 6 : Mise en évidence de l'effet de l'agent actif selon l'exemple 1 sur la morphologie de l'épiderme humain adulte

Le but de cette étude est d'analyser la morphologie de l'épiderme adulte humain afin de déterminer l'effet de l'agent actif selon l'exemple 1 sur la qualité des structures cutanées.

### Protocole :

Des biopsies de peau humaine sont mises en culture à l'interface air/liquide et traitées par application topique avec une solution à 1 % ou 3% d'agent actif selon l'exemple 1, pendant 24 heures. Les biopsies de peau sont ensuite incluses dans de la paraffine et des coupes histologiques de 3 µm d'épaisseur sont réalisées. Les coupes sont déposées sur des lames Superfrost Plus (Menzel Gläser, Thermo Scientific), puis déparaffinées dans le xylène et réhydratées dans une série de solutions alcool-eau. Les coupes sont ensuite colorées par de l'hématoxyline à 50 % pendant 3 minutes, rincées, puis colorées à l'éosine 60 %, pendant 3 minutes et rincées à l'eau. Les coupes sont ensuite déshydratées, montées dans l'Eukitt et examinées en microscopie optique.

### Résultats :

Les coupes histologiques de peau traitées par l'agent actif montrent que la couche cornée apparait plus épaisse et plus cohésive. On observe également une plus grande densité des cellules de la couche basale, qui apparaissent mieux orientées dans l'axe vertical et plus homogènes.

### Conclusions :

L'agent actif selon l'exemple 1 améliore la morphologie de l'épiderme dans son ensemble.

### Exemple 6 : Préparation de compositions

**1 - Crème de jour**

| ***Noms commerciaux*** | ***Noms INCI*** | ***% massique*** |
|---|---|---|
| ***PHASE A*** | | |
| MONTANOV 68 | Cetearyl Alcohol (and) Cetearyl Glucoside | 5,00 |
| HUILE DE JOJOBA | Simmondsia Chinensis (Jojoba) Seed Oil | 3,00 |
| HUILE DE VASELINE | Paraffinum Liquidum (Mineral Oil) | 2,00 |
| SQUALANE | Squalane | 3,00 |
| CERAPHYL 368 | Ethylhexyl palmitate | 4,00 |
| CERAPHYL 41 | C12-C15 Alkyl Lactate | 3,00 |
| RAPITHIX A-60 | Sodium polyacrylate (and) Hydrogenated Polydecene (and) Trideceth -6 | 0,30 |

| ***PHASE B*** | | |
|---|---|---|
| GLYCERINE | Glycerin | 5,00 |
| ALLANTOÏNE | Allantoin | 0,10 |
| EAU DEMINERALISEE | Aqua (Water) | qsp 100 |

| ***PHASE C*** | | |
|---|---|---|
| ROKONSAL MEP | Phenoxyethanol (and) Methylparaben (and) Ethylparaben (and) Propylparaben | 0,50 |

| ***PHASE D*** | | |
|---|---|---|
| AGENT ACTIF SELON EXEMPLE 1 | | 5 % |

| ***Noms commerciaux*** | ***Noms INCI*** | ***% massique*** |
|---|---|---|
| ***PHASE E*** | | |
| PARFUM | Parfum (Fragrance) | qsp |

### Mode Opératoire :

Peser les ingrédients de la phase grasse et chauffer à 70°C sous agitation. Préparer la phase B et la chauffer à 70°C. Emulsionner la phase A dans la phase B. Ajouter la phase D vers 50°C sous agitation. En dessous de 40°C, additionner l'agent actif (Phase D). Parfumer et refroidir jusqu'à température ambiante.

**2 - Crème hydratante W/O**

| ***Noms commerciaux*** | ***Noms INCI*** | ***% massique*** |
|---|---|---|
| ***PHASE A*** | | |
| ARLACEL P 135 | PEG-30 Dipolyhydroxystearate Isononanoate | 2,00 |
| CERAPHYL 375 | Isostearyl Neopentanoate | 3,00 |
| PANALANE L-14E | Hydrogenated Polyisobutene | 3,00 |
| CERAPHYL ODS | Octyldodecyl Stearate | 9,00 |
| CERAPHYL 368 | Ethylhexyl Palmitate | 3,00 |

| ***PHASE B*** | | |
|---|---|---|
| EAU DEMINERALISEE | Aqua (Water) | qsp 100 |
| ATLAS G-2330 | Sorbeth-30 | 4,00 |
| SULFATE DE MAGNESIUM 7 H2O | Magnésium Sulfate | 0,70 |
| AGENT ACTIF SELON EXEMPLE 1 | | 2 % |

| ***PHASE C*** | | |
|---|---|---|
| LIQUAPAR OPTIMA | Phenoxyethanol (and) Methylparaben (and) Isopropylparaben (and) Isobutylparaben (and) Butylparaben | 0,50 |

| ***PHASE D*** | | |
|---|---|---|
| PARFUM | Parfum (Fragrance) | qsp |

### Mode Opératoire :

Peser la phase A et chauffer à 75°C sous agitation. Préparer la phase B et la chauffer à 75°C. Emulsionner la phase B dans la phase A sous forte agitation rotor-stator.

Homogénéiser quelques minutes. Refroidir brutalement à l'aide d'un bain d'eau glacée sous vive agitation. Ajouter la phase C aux environs de 50°C et additionner le parfum (phase D) à 40°C. Poursuivre le refroidissement jusqu'à température ambiante.

**3 - Lotion hydratante**

| ***Noms commerciaux*** | ***Noms INCI*** | ***% massique*** |
|---|---|---|
| EAU DEMINERALISEE | Aqua (Water) | qsp 100 |
| GLYCERINE | Glycerin | 2,00 |
| PROPYLENE GLYCOL | Propylene Glycol | 2,00 |
| GLUCAM E-10 | Methyl Gluceth -10 | 1,00 |
| NEOSORB | Sorbitol | 5,00 |
| ALLANTOINE | Allantoin | 0,10 |
| ROKONSAL BSB | Benzoic Acid (and) Sorbic Acid (and) Benzyl Alcohol | 0,30 |
| AGENT ACTIF SELON EXEMPLE 1 | | 0,5 % |
| PARFUM hydrosoluble | Parfum (Fragrance) | qsp |

### Mode Opératoire :

Incorporer les ingrédients un à un à la quantité d'eau nécessaire et agiter jusqu'à parfaite dissolution. Réajuster le pH aux environs de 5,5 si nécessaire. Incorporer l'actif en fin de formulation. Parfumer avec un parfum hydrosoluble sous faible agitation.

## Revendications

1. Extrait peptidique de germe de caroube (*Ceratonia siliqua L.*) activateur de l'expression des aquaporines, **caractérisé en ce qu'**il est obtenu après hydrolyse et **en ce que** les composés de nature peptidique qu'il contient sont des peptides de poids moléculaire inférieur à 5 kDa et que sa teneur en polyphénols est inférieure à 1 %.

2. Extrait peptidique selon la revendication 1, **caractérisé en ce qu'**il est solubilisé dans un ou plusieurs solvants physiologiquement acceptables, choisi parmi l'eau, le glycérol, l'éthanol, le propanediol, le butylène glycol, le dipropylène glycol, les diglycols éthoxylés ou propoxylés, les polyols cycliques ou tout mélange de ces solvants.

3. Extrait peptidique selon l'une des revendications 1 ou 2, **caractérisé en ce qu'**il contient entre 1,5 et 3,5 g/l de composés de nature peptidique.

4. Extrait peptidique selon l'une des revendications précédentes, **caractérisé en ce qu'**il contient entre 0,1 et 0,3 g/l de sucres.

5. Composition cosmétique comprenant, dans un milieu physiologiquement acceptable, en tant qu'agent actif activateur de l'expression des aquaporines, l'extrait peptidique défini selon l'une des revendications précédentes, à une concentration comprise entre 0,0001 % et 20 % du poids total de la composition, et comprenant au moins un autre agent actif choisi parmi le glycérol, la vitamine C, la vitamine E, le coenzyme Q10, les rétinoïdes.

6. Composition cosmétique selon la revendication 5, **caractérisée en ce qu'**elle comprend l'extrait peptidique défini selon l'une des revendications 1 à 4, à une concentration comprise entre 0,05 % et 5 % du poids total de la composition.

7. Composition selon la revendication 5 ou 6, **caractérisée en ce qu'**elle se présente sous une forme adaptée à l'application par voie topique.

8. Composition pharmaceutique comprenant, dans un milieu physiologiquement acceptable, l'extrait peptidique défini selon l'une des revendications 1 à 4, pour son utilisation comme médicament.

9. Composition pharmaceutique selon la revendication 8 pour son utilisation dans une méthode de traitement des sécheresses pathologiques de la peau ou des muqueuses, choisies parmi l'eczéma, la xérose, les dermatites atopiques, les sécheresses buccales, oculaires ou vaginales.

10. Utilisation non-thérapeutique d'un extrait peptidique de germe de caroube (*Ceratonia siliqua L.*) selon l'une des revendications 1 à 4, en tant qu'agent actif hydratant.

11. Utilisation non-thérapeutique d'un extrait peptidique de germe de caroube (*Ceratonia siliqua L.*) selon l'une des revendications 1 à 4, pour protéger la peau et les muqueuses de la déshydratation.

12. Utilisation non-thérapeutique d'un extrait peptidique de germe de caroube (*Ceratonia siliqua L.*) selon l'une des revendications 1 à 4, pour régénérer et améliorer l'effet barrière de l'épiderme.

13. Utilisation non-thérapeutique d'un extrait peptidique de germe de caroube (*Ceratonia siliqua L.*) selon l'une des revendications 1 à 4, pour lutter de manière préventive et/ou curative contre les rides et les ridules.

14. Utilisation non-thérapeutique d'un extrait peptidique de germe de caroube (*Ceratonia siliqua L.*) selon l'une des revendications 1 à 4, pour lutter de manière préventive et/ou curative contre la perte d'élasticité et de fermeté de la peau.

## Patentansprüche

1. Johannisbrotkeim-Peptidextrakt (*Ceratonia siliqua L.*)*,* der die Expression der Aquaporine aktiviert, **dadurch gekennzeichnet, dass** er nach Hydrolyse erhalten wird und dadurch, dass die Verbindungen von Peptidnatur, die er enthält, Peptide mit einem Molekulargewicht von unter 5 kDa sind und dass sein Gehalt an Polyphenolen unter 1 % beträgt.

2. Peptidextrakt nach Anspruch 1, **dadurch gekennzeichnet, dass** er in einem oder mehreren physiologisch verträglichen Lösungsmitteln gelöst ist, ausgewählt aus Wasser, Glycerin, Ethanol, Propandiol, Butylenglycol, Dipropylenglycol, ethoxylierten oder propoxylierten Diglycolen, zyklischen Polyolen oder jedem Gemisch dieser Lösungsmittel.

3. Peptidextrakt nach einem der Ansprüche 1 oder 2, **dadurch gekennzeichnet, dass** er zwischen 1,5 und 3,5 g/l an Verbindungen von Peptidnatur enthält.

4. Peptidextrakt nach einem der vorhergehenden Ansprüche, **dadurch gekennzeichnet, dass** der zwischen 0,1 und 0,3 g/l an Zuckern enthält.

5. Kosmetische Zusammensetzung, die in einem physiologisch verträglichen Medium als Wirkstoff, der die Expression der Aquaporine aktiviert, den definierten Peptidextrakt nach einem der vorhergehenden Ansprüche in einer Konzentration im Bereich zwischen 0,0001 % und 20 % des Gesamtgewichts der Zusammensetzung umfasst, und wenigstens einen weiteren Wirkstoff, ausgewählt aus Glycerin, Vitamin C, Vitamin E, Coenzym Q10 und Retinoiden umfasst.

6. Kosmetische Zusammensetzung nach Anspruch 5, **dadurch gekennzeichnet, dass** sie den definierten Peptidextrakt nach einem der Ansprüche 1 bis 4 in einer Konzentration im Bereich zwischen 0,05 % und 5 % des Gesamtgewichts der Zusammensetzung umfasst.

7. Zusammensetzung nach Anspruch 5 oder 6, **dadurch gekennzeichnet, dass** sie sich in einer Form zeigt, die für die topische Anwendung geeignet ist.

8. Pharmazeutische Zusammensetzung, die in einem physiologisch verträglichen Medium den definierten Peptidextrakt nach einem der Ansprüche 1 bis 4 umfasst, für ihre Verwendung als Arzneimittel.

9. Pharmazeutische Zusammensetzung nach Anspruch 8 für ihre Verwendung in einem Verfahren zur Behandlung der pathologischen Trockenheiten der Haut oder der Schleimhäute, ausgewählt aus Ekzem, Xerose, atopischer Dermatitis, Mundtrockenheit, Augentrockenheit oder vaginaler Trockenheit.

10. Nicht-therapeutische Verwendung eines Johannisbrotkeim-Peptidextrakts (*Ceratonia siliqua L.*) nach einem der Ansprüche 1 bis 4 als hydratisierender Wirkstoff.

11. Nicht-therapeutische Verwendung eines Johannisbrotkeim-Peptidextrakts (*Ceratonia siliqua L.*) nach einem der Ansprüche 1 bis 4, um die Haut und die Schleimhäute vor der Austrocknung zu schützen.

12. Nicht-therapeutische Verwendung eines Johannisbrotkeim-Peptidextrakts (*Ceratonia siliqua L.*) nach einem der Ansprüche 1 bis 4, um die Barrierewirkung der Epidermis zu regenerieren und zu verbessern.

13. Nicht-therapeutische Verwendung eines Johannisbrotkeim-Peptidextrakts (*Ceratonia siliqua L.*) nach einem der Ansprüche 1 bis 4, um in präventiver und/oder kurativer Weise die Falten und die Fältchen zu bekämpfen.

14. Nicht-therapeutische Verwendung eines Johannisbrotkeim-Peptidextrakts (*Ceratonia siliqua L.*) nach einem der Ansprüche 1 bis 4, um in präventiver und/oder kurativer Weise den Elastizitäts- und Festigkeitsverlust der Haut zu bekämpfen.

## Claims

1. Peptide extract of carob germ *(Ceratonia siliqua L)* activator of the expression of aquaporins, **characterised in that** it is obtained after hydrolysis and **in that** the peptide type compounds contained in it are peptides with a molecular weight of less than 5 kDa and that its content of polyphenols is less than 1%.

2. Peptide extract according to claim 1, **characterised in that** it is solubilised in one or several physiologically acceptable solvents chosen from among water, glycerol, ethanol, propanediol, butylene glycol, dipropylene glycol, ethoxylated or propoxylated diglycols, cyclic polyols or any mix of these solvents.

3. Peptide extract according to either claim 1 or 2, **characterised in that** it contains between 1.5 and 3.5 g/l of peptide type compounds.

4. Peptide extract according to one of the previous claims, **characterised in that** it contains between 0.1 and 0.3 g/l of sugars.

5. Cosmetic composition in a physiologically acceptable medium as an activating agent for the expression of aquaporins, the peptide extract defined according to one of the previous claims, with a concentration of between 0.0001% and 20% of the total weight of the composition and comprising at least one other active agent chosen from among glycerol, vitamin C, vitamin E, coenzyme Q10 and retinoids.

6. Cosmetic composition according to claim 5, **characterised in that** it comprises the peptide extract defined according to one of claims 1 to 4, with a concentration of between 0.05% and 5% of the total weight of the composition.

7. Cosmetic composition according to claim 5 or 6, **characterised in that** it is a form adapted to topical application.

8. Pharmaceutical composition comprising the peptide extract defined according to one of claims 1 to 4 in a physiologically acceptable medium, for use as a medicine.

9. Pharmaceutical composition according to claim 8, for use in a method of treatment of pathological dryness of the skin or mucous membranes, chosen from among eczema, xerosis, atopic dermatitis, dryness of the mouth, eyes or the vagina.

10. Non-therapeutic use of a peptide extract of carob germ *(Ceratonia siliqua L.)* according to one of claims 1 to 4 as a moisturising agent.

11. Non-therapeutic use of a peptide extract of carob germ *(Ceratonia siliqua L.)* according to one of claims 1 to 4 to protect the skin and mucous membrane from dehydration.

12. Non-therapeutic use of a peptide extract of carob germ *(Ceratonia siliqua L.)* according to one of claims 1 to 4 to regenerate and improve the barrier effect of the epidermis.

13. Non-therapeutic use of a peptide extract of carob germ (*Ceratonia siliqua L.*) according to one of claims 1 to 4 for preventive and/or remedial treatment of fine lines and wrinkles.

14. Non-therapeutic use of a peptide extract of carob germ (*Ceratonia siliqua L.*) according to one of claims 1 to 4 for preventive and/or remedial treatment of loss of elasticity and firmness of the skin.
